# EUROPÄISCHE PATENTANMELDUNG

(11) **EP 4 681 742 A2**
(43) Veröffentlichungstag der Anmeldung: **21.01.2026**
(21) Anmeldenummer: 25215095.8
(22) Anmeldetag: 28.07.2020
(51) Int. Cl.: A61L 2/03

(54) **BEHANDLUNGSSYSTEM MIT EINEM KONTAKTIERUNGSSYSTEM**

(30) Priorität: 30.07.2019 DE 102019211370
(62) Teilanmeldung aus: 20757538.2
(71) Anmelder: Institut Straumann AG, 4002 Basel (CH)
(72) Erfinder: BRODBECK, Urs, 8703 Erlenbach (CH); EBERLE, Roger, 9445 Rebstein (CH); JAENECKE, Bernd, 9436 Balgach (CH)
(74) Vertreter: Tergau & Walkenhorst Intellectual Property GmbH

(57) **Zusammenfassung**

Ein Kontaktierungssystem (20) zur elektrischen Kontaktierung von in einen isolierenden Mantel (10) eines Kabelstrangs (4a) eingebetteten elektrischen Leiterelementen (14) soll auf besonders einfache und kostengünstige Weise eine auch hohen Zuverlässigkeitsanforderungen genügende elektrische Kontaktierung des Kabelstrangs (4a) ermöglichen. Dies wird erfindungsgemäß erreicht mit einem Anschlussmodul (22), in dessen Außengehäuse (25) ein Teilabschnitt des Kabelstrangs (4a) fixierbar ist, und mit einer Anzahl von Kontaktstiften (36), die von der Seite her relativ zur Längsrichtung des Kabelstrangs (4a) gesehen quer durch das Außengehäuse (25) hindurchgeführt sind.

## Beschreibung

Die Erfindung betrifft ein Kontaktierungssystem zur elektrischen Kontaktierung von in einen isolierenden Mantel eines Kabelstrangs eingebetteten elektrischen Leiterelementen. Sie betrifft weiter die Verwendung eines solchen Kontaktierungssystems.

Aus der WO 2014/075755 A1, der WO 2014/122187 A1, der WO 2014/122188 A1 und der WO 2016/023998 A1, deren Offenbarungen voll umfänglich mit einbezogen werden ("incorporation by reference"), ist jeweils ein Behandlungselement, insbesondere zur Verwendung mit einem Implantat-Teil, sowie ein Verfahren zum Reinigen eines DentalImplantat-Teils bekannt. Eine derartige Reinigung eines Implantat-Teils kann wünschenswert oder erforderlich sein, um den Erhalt des inserierten Implantats in der Knochensubstanz zu gewährleisten. An der von Gewebe und Gewebeflüssigkeit umschlossenen festen Oberfläche von Implantaten kann sich nämlich ein Biofilm bilden, der Bakterien beinhaltet, die letztlich zu chronischen und wiederkehrenden Infektionen führen können. Dieses Erkrankungsbild wird als Periimplantitis bezeichnet. Insbesondere im dentalen Bereich ist ähnlich wie bei der Parodontitis eine Kombination aus vernachlässigter Mundhygiene, Anhaftung von Biofilm an der üblicherweise mikrorauen Oberfläche des Dentalimplantats und weiterer Faktoren ursächlich für das Vollbild der Periimplantitis, welche sich durch eine zunehmende Belastung und Zerstörung des Hart- und Weichgewebes auszeichnet. Die Bereiche, an denen sich das Hart- und/oder Weichgewebe zurückzieht, werden dabei in der Regel mit einem Biofilm überzogen.

Das in den genannten Anmeldungen beschriebene Reinigungsverfahren beruht auf dem Konzept, den die Verunreinigung bildenden Biofilm bzw. die Keime ausgehend von der Implantatoberfläche abzutöten und zu entfernen, ohne dabei die Implantatoberfläche zu beschädigen. Dazu ist ein elektrolytischer Prozess vorgesehen, bei dem Ionen (Kationen und/oder Anionen) mittels elektrostatischer Kräfte durch den Biofilm hindurch befördert werden. Diese Ionen reagieren an der Implantatoberfläche chemisch oder elektrochemisch. Durch diese Reaktionen werden neue Stoffverbindungen geschaffen und/oder die Ionen selbst und/oder Teile dieser Ionen in den atomaren Zustand überführt. Darüber hinaus besteht zudem die Möglichkeit, dass die Ionen mit dem Oberflächenmaterial reagieren (z. B. Bildung einer Oxidschicht oder Materialabtrag). Dieser Prozess bewirkt zum Einen aufgrund der gebildeten chemischen Substanzen eine Keimabtötung, zum Anderen aber auch die Bildung von Gasblasen, die den Biofilm auf mechanische Weise abtragen.

Die keimtötende Wirkung dieses Prozesses basiert auf unterschiedlichen Effekten. Zum einen werden durch das Anlegen einer elektrischen Spannung Ionen aus dem Biofilm selbst (auch aus den Bakterien) zur Anode oder Kathode befördert. Dies kann zur Abtötung von Bakterien und Viren führen. Darüber hinaus können die Ionen, während sie den Biofilm passieren, biochemische Reaktionen eingehen, was ebenfalls zur Abtötung von Bakterien und/oder Viren führen kann. Eine weitere Möglichkeit der Abtötung besteht darin, dass die an der Implantatoberfläche neu gebildeten Stoffverbindungen antibakterielle und/oder antivirale und/oder antifungizide Wirkung besitzen. Dies kann natürlich auch passieren, wenn die Ionen in den atomaren Zustand übergehen.

Das in den genannten Anmeldungen beschriebene Behandlungselement ist spezifisch dafür ausgelegt, dieses Reinigungsverfahren direkt am inserierten Dentalimplantat durchzuführen, also bevorzugt während sich das Pfostenteil im Knochen im Patientenmund befindet. Dazu ist das Behandlungselement dazu vorgesehen, direkt mit dem inserierten Pfostenteil verbunden zu werden und sodann eine geeignete Behandlungsflüssigkeit, die bei Beaufschlagung mit elektrischem Strom als Basis für den angestrebten elektrolytischen Prozess dienen kann, in unmittelbarer Nähe zum inserierten Pfostenteil in den betroffenen Raumbereich der benachbarten Knochensubstanz auszubringen und mit dem elektrischen Strom zu beaufschlagen. Zum Einsatz dieses Behandlungselements ist somit die Herstellung eines sowohl mechanischen als auch elektrischen Kontakts mit dem inserierten Pfostenteil erforderlich. Hierzu muss bei der in der genannten Anmeldung beschriebenen Bauweise des Behandlungselements zum Zweck seiner Fixierung am Pfostenteil in der Regel die Prothetik am Dentalimplantat und gegebenenfalls auch dessen Abutment vorübergehend entfernt werden.

Der vorliegenden Erfindung liegt die Aufgabe zu Grunde, ein Kontaktierungssystem der oben genannten Art anzugeben, das auf besonders einfache und kostengünstige Weise eine auch hohen Zuverlässigkeitsanforderungen genügende elektrische Kontaktierung eines zur Verwendung mit einem Behandlungssystem der genannten Art vorgesehenen Kabelstrangs ermöglicht.

Diese Aufgabe wird erfindungsgemäß gelöst mit einem Anschlussmodul, in dessen Au-ßengehäuse ein Teilabschnitt des Kabelstrangs fixierbar ist, und mit einer Anzahl von Kontaktstiften, die von der Seite her relativ zur Längsrichtung des Kabelstrangs gesehen quer durch das Außengehäuse hindurchgeführt sind.

Vorteilhafte Ausgestaltungen der Erfindung sind Gegenstand der Unteransprüche. Weitere und/oder alternative vorteilhafte Ausgestaltungen der Erfindung ebenso wie weitere als eigenständige Erfindungen angesehene Ausgestaltungen ergeben sich auch aus der Figurenbeschreibung.

Die Erfindung geht von der Überlegung aus, dass gerade bei der Verwendung in einem Behandlungssystem der oben genannten Art der Kabelstrang mit besonders einfach gehaltenen und insbesondere auch vom nicht weiter geschulten Benutzer ohne weiteres nutzbaren Mitteln kontaktierbar sein sollte. Um hierbei aber dennoch die üblicherweise gerade bei Anwendungen im medizinischen Bereich vergleichsweise hohen Anforderungen an die Zuverlässigkeit der Kontaktierung zu erfüllen, sollte das Kontaktierungssystem bei einfacher Handhabbarkeit besonders fehlertolerant ausgeführt sein. Um dies zu ermöglichen, ist eine Kontaktierung der Leiterelemente im Kabelstrang durch Kontaktstifte vorgesehen, die in der Art einer "kreuzenden Leitungsführung" mit ihrer Längsrichtung quer zur Längsrichtung des Kabelstrangs und somit der in diesem geführten Leiterelemente ausgerichtet sind. Damit kann eine zuverlässige Kontaktierung durch jeweilige Ausbildung eines Kontaktpunkts zwischen Kontaktstift einerseits und zugeordnetem Leiterelement andererseits erreicht werden, ohne dass eine genaue Positionierung von Kabelstrang oder Kontaktstiften, jeweils in ihrer Längsrichtung gesehen, notwendig wäre.

Vorteilhafterweise sind zudem die Kontaktstifte relativ zueinander derart voneinander beabstandet positioniert, dass der im Teilabschnitt abisolierte Kabelstrang gerade, besonders bevorzugt leicht klemmend, zwischen die Kontaktstifte passt. Damit kann einerseits in der Art einer Doppelfunktion durch die Kontaktstifte zusätzlich zur eigentlichen elektrischen Kontaktierung auch die mechanische Fixierung des Teilabschnitts des Kabelstrangs im Außengehäuse des Anschlussmoduls bewirkt oder zumindest unterstützt werden. Andererseits wird dadurch aber auch ein besonders inniger Kontakt zwischen dem jeweiligen Kontaktstift und dem zugeordneten Leiterelement hergestellt, so dass die Zuverlässigkeit der elektrischen Verbindung noch weiter erhöht wird.

Ganz besonders vorteilhaft wird das Kontaktsystem in einem Behandlungssystem der oben genannten Art verwendet.

Ein Ausführungsbeispiel der Erfindung wird anhand einer Zeichnung näher erläutert. Darin zeigen:
- FIG. 1: ein Behandlungssystem zur Reinigung eines mit Biofilm verunreinigten Bauteils,
- FIG. 2: einen Verbindungsschlauch des Behandlungssystems gemäß FIG. 1 in perspektivischer Ansicht,
- FIG. 3: den Verbindungsschlauch gem. FIG. 2 im Längsschnitt,
- FIG. 4: den Verbindungsschlauch gem. FIG. 2 in zwei Varianten im Querschnitt,
- FIG. 5: ein Kontaktierungssystem des Behandlungssystems gemäß FIG. 1 in seitlicher Ansicht,
- FIG. 6: ein Anschlussmodul des Kontaktierungssystems gem. FIG. 5 mit angeschlossenem Verbindungsschlauch in seitlicher Ansicht,
- FIG. 7: das Anschlussmodul gem. FIG. 6 in Draufsicht,
- FIG. 8: das Anschlussmodul gem. FIG. 6 in perspektivischer Ansicht,
- FIG. 9: das Anschlussmodul gem. FIG. 6 mit getrennten Gehäusehälften in verschiedenen Ansichten,
- FIG. 10: das Anschlussmodul gem. FIG. 6 im Längsschnitt,
- FIG. 11: einen vergrößerten Ausschnitt aus FIG. 10,
- FIG. 12: die für die Herstellung eines elektrischen Kontakts vorgesehenen Komponenten,
- FIG. 13: einen Behandlungskopf des Behandlungssystems gemäß FIG. 1 im Längsschnitt,
- FIG. 14: den Behandlungskopf gem. FIG. 13 im Längsschnitt mit angeschlossenem Verbindungsschlauch,
- FIG. 15: das Außengehäuse des Behandlungskopfs gem. FIG. 13 in zwei perspektivischen Ansichten,
- FIG. 16: einen Verbindungsstift des Behandlungskopfs gem. FIG. 13 in perspektivischer Ansicht,
- FIG. 17: einen Isolatorkörper,
- FIG. 18: den in den Isolatorkörper gem. FIG. 17 eingesteckten Verbindungsstift gem. FIG. 16 in perspektivischer Ansicht, und
- FIG. 19: den in den Isolatorkörper gem. FIG. 17 eingesteckten Verbindungsstift gem. FIG. 16 im Längsschnitt.

Gleiche Teile sind in allen Figuren mit denselben Bezugszeichen versehen.

Generell besteht bei Dental-Implantatsystemen, insbesondere auch bei zweiteiligen Implantatsystemen, und möglicherweise auch bei anderen medizinischen Implantaten das Problem, dass durch ein Eindringen von Bakterien oder Keimen in den Gewebebereich in der Nähe der Insertionsstelle, insbesondere im Bereich des in den Kiefer eingebrachten Außengewindes, Entzündungen oder Entzündungsherde entstehen können. Derartige, insbesondere auch in Folge einer so genannten Periimplantitis entstehende Entzündungen können, insbesondere wenn sie sich über einen längeren Zeitraum entwickeln und verfestigen können, zu einer gravierenden Beeinträchtigung des Gewebes und des Knochens im Bereich der Insertionsstelle führen. Ohne geeignete Gegenmaßnahmen können diese Beeinträchtigungen dazu führen, dass das gesamte Implantatsystem wieder aus dem Knochen entfernt und nach einem Knochenaufbau erneut mit einem Implantatsystem versehen oder durch andere Prothetik ersetzt werden muss. Dieser durch die Periimplantitis hervorgerufene, äußerst unerwünschte Effekt kann somit zu einem Totalverlust des Implantatsystems führen, so dass erneute chirurgische Maßnahmen wie beispielsweise ein Ausschaben des betroffenen Bereichs im Kieferknochen und die Neuversorgung mit einem Implantatsystem notwendig werden können. Durch eine derartige Entnahme kann es zudem zu Knochenverlust oder sonstigem Verlust an Gewebesubstanz kommen, die im Extremfall soweit führen kann, dass eine Neuversorgung mit einem anderen Implantat gar nicht mehr möglich ist. Eine derartige durch Periimplantitis hervorgerufene Notwendigkeit einer Neuversorgung kann auch nach vergleichsweise langen Zeiträumen nach dem ersten Einsetzen des Implantatsystems von beispielsweise bis zu einigen Jahren oder sogar Jahrzehnten auftreten.

Die im Zusammenhang mit einer Periimplantitis beobachteten Keime oder Bakterien können dabei grundsätzlich das Innere der Komponenten des Implantats besiedeln, haften aber in der Regel bevorzugt direkt an der Oberfläche des in den Kieferknochen inserierten Dentalimplantats im Kontaktbereich mit dem umgebenden Gewebe oder Knochenmaterial, also insbesondere im Bereich des Außengewindes, an. In dessen Bereich kann die Oberfläche des Dentalimplantats mit einer Aufrauhung oder dergleichen versehen sein, um das Einwachsen in das Gewebe oder den Knochen besonders zu begünstigen und das Einheilen des Dentalimplantats nach der Insertion zu unterstützen. Gerade im Bereich einer derartigen, eigentlich als besonders günstig für das Implantatsystem angesehenen Aufrauhung der Oberfläche kann aber die Ansiedlung der Keime oder Bakterien vermehrt stattfinden, wobei die Rauigkeit ein gezieltes Entfernen der vorhandenen Keime oder Bakterien noch zusätzlich erschwert.

Es besteht daher der dringende Wunsch nach geeigneten Gegenmaßnahmen, um für den Fall einer sich anbahnenden oder bereits aufgetretenen Periimplantitis unter Erhaltung des bereits eingesetzten Implantatsystems wirksam den Entzündungsherd bekämpfen und die eingedrungenen Keime abtöten und /oder entfernen zu können, so dass sich anschließend wieder gesundes Gewebe oder gesunde Knochensubstanz im Bereich um das Außengewinde herum bilden kann. Dazu ist wünschenswert, zusätzlich zu einem gezielten Abtöten der Keime oder Bakterien im betroffenen Bereich auch noch deren Materialreste und Fragmente zuverlässig aus dem betroffenen Raumbereich zu entfernen, so dass anschließend der betroffene Bereich wieder von gesundem Gewebe oder Knochenmaterial ausgefüllt werden und sich wieder eine innige Verbindung zwischen der Außenoberfläche des Dentalimplantats und dem umgebenden Gewebe oder Knochenmaterial bilden kann. Zudem sollte der von der Bakterienbeschichtung gebildete Biofilm einschließlich der organischen Reste abgetöteter Bakterien zuverlässig entfernt werden.

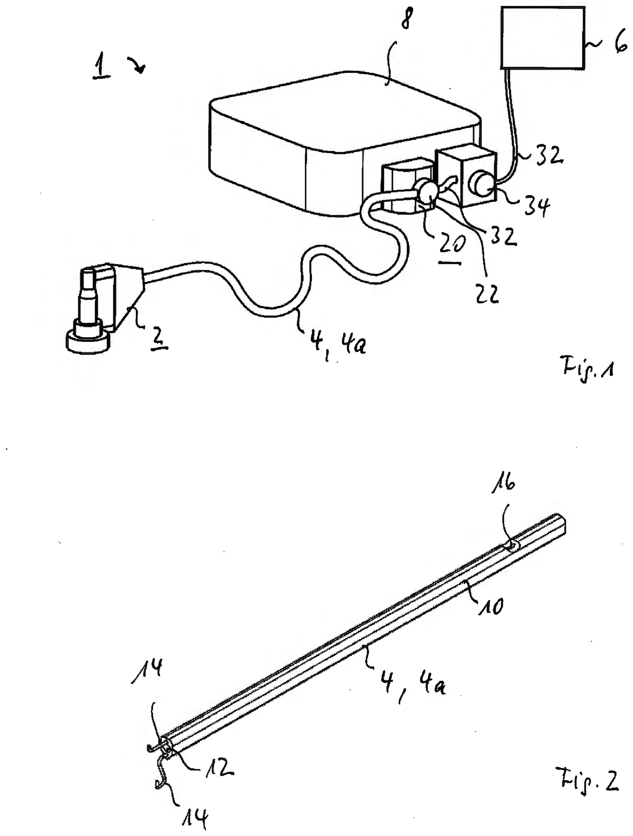

Zu diesem Zweck, also zum Abtöten und/oder mechanischen Ablösen von Keimen oder Bakterien im Insertionsbereich des Dentalimplantats und insbesondere auch zum anschließenden Ausspülen, Entfernen und Austragen der Gewebe- und Materialreste der abgetöteten Bakterien, ist das in FIG. 1 dargestellte Behandlungssystem 1 vorgesehen. Dieses beruht hinsichtlich seiner Ausgestaltung und prinzipiellen Ausführung auf zwei jeweils als eigenständig erfinderisch angesehenen Grundkonzepten: Einerseits ist es als primäres Auslegungsziel dafür ausgestaltet, eventuell an der Oberfläche des Dentalimplantats, insbesondere im Bereich Außengewindes, noch anhaftende Reste oder Fragmente von Keimen und/oder Bakterien durch eine geeignete Beaufschlagung mit Strom oder Stromstößen von der Außenoberfläche des Dentalimplantats abzulösen, so dass sie anschließend ausgewaschen werden können. Andererseits ist es zusätzlich auch dafür ausgelegt, die im Insertionsbereich des Implantats vorhandenen Keime oder Bakterien durch gezielte Zuführung eines bakterioziden, aber für den menschlichen Organismus verträglichen Reinigungs- oder Desinfektionsmittels gezielt abzutöten.

Das Behandlungssystem 1 gemäß der Darstellung in FIG. 1 ist dementsprechend zur Reinigung eines mit Biofilm verunreinigten Bauteils, insbesondere eines Implantat-Teils, mit einem elektrolytischen Reinigungskonzept vorgesehen, wie es beispielsweise aus den Druckschriften WO 2014/075755 A1, der WO 2014/122187 A1, der WO 2014/122188 A1 und der WO 2016/023998 A1 bekannt ist. Das Behandlungssystem 1 ist somit dafür ausgelegt, das behandlungsbedürftige Bauteil mit einer spezifischen, geeignet gewählten Behandlungsflüssigkeit zu beaufschlagen und dann oder dabei einen Stromfluss durch das behandlungsbedürftige Bauteil und die Behandlungsflüssigkeit zu erzeugen. Dazu umfasst das Behandlungssystem 1 einen Behandlungskopf 2, der mechanisch mit dem behandlungsbedürftigen Bauteil verbunden, beispielsweise auf dieses aufgesteckt, aufgeschraubt oder aufgedrückt werden kann, und über den sowohl die Behandlungsflüssigkeit auf das behandlungsbedürftige Bauteil ausgebracht als auch die elektrische Kontaktierung zur Einstellung des gewünschten Stromflusses vorgenommen werden kann. Der Behandlungskopf 2 ist über einen Verbindungsschlauch 4 sowohl medienseitig mit einem Vorratsbehälter 6 für die Behandlungsflüssigkeit als auch elektrisch mit einer als Strom- oder Spannungsquelle zur Einstellung des gewünschten Stromflusses vorgesehenen elektrischen Versorgungseinheit 8 verbunden.

Das Behandlungssystem 1 ist dabei unter anderem gezielt für eine besonders erleichterte und zuverlässige Handhabung ausgelegt. Um dies besonders zu begünstigen, ist der Verbindungsschlauch 4 in der Art eines integrierten Bauteils sowohl zur medienseitigen als auch zur elektrischen Verbindung des Behandlungskopfs 2 mit dem Vorratsbehälter 6 bzw. der Versorgungseinheit 8 vorgesehen. Damit ist es beim Einsatz des Behandlungssystems 1 nicht notwendig, mehrere verschiedene Anschlussschläuche, Verbindungsdrähte oder dergleichen zu bedienen und zu koordinieren.

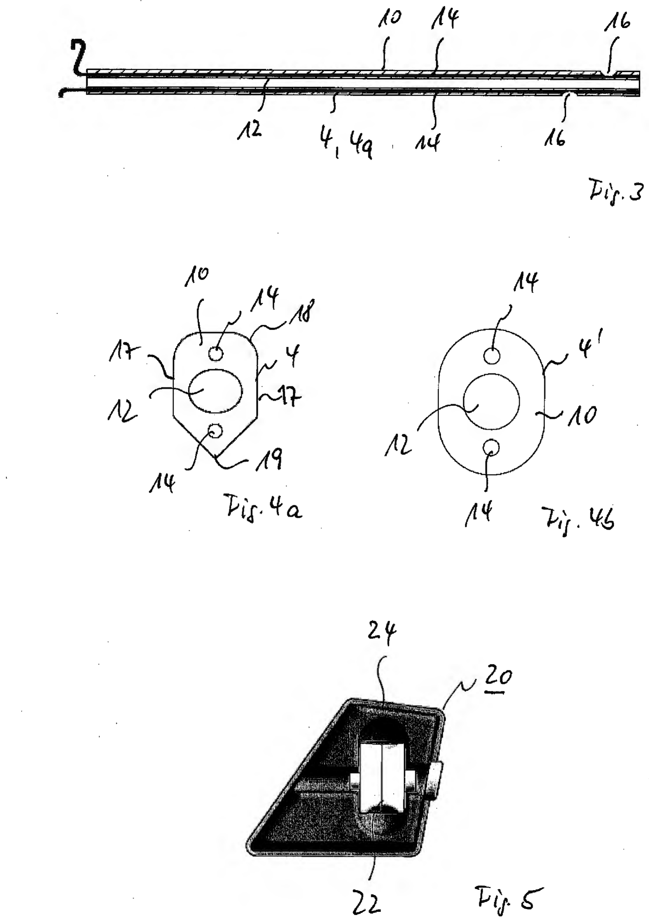

Der Verbindungsschlauch 4 ist in FIG. 2 in perspektivischer Ansicht, in FIG. 3 im Längsschnitt und in FIG. 4 im Querschnitt in zwei Ausführungsformen mit jeweils ganz besonders bevorzugter, jeweils als eigenständig erfinderisch angesehener Querschnittskontur (FIG. 4a bzw FIG. 4b) gezeigt. Im Sinne der oben genannten Ausführung als integriertes Bauteil wird der Verbindungsschlauch 4 im Wesentlichen durch einen Mantel 10 aus einem geeignet gewählten Schlauchmaterial, besonders bevorzugt PVC, TPU oder Silikon, gebildet. Der Mantel 10 weist in seinem Inneren einen Medienkanal 12 auf, durch den die Behandlungsflüssigkeit vom Vorratsbehälter 6 zum Behandlungskopf 2 strömen kann. In den Mantel 10 sind zudem eine Anzahl von, im Ausführungsbeispiel zwei, elektrische Leitungselemente 14 integriert. Im Hinblick auf die vorgesehenen Funktionen des Verbindungsschlauchs 4 liegen als Auslegungskriterien für die Auswahl des Mantelmaterials besonders bevorzugt einerseits eine ausreichende Inertheit gegenüber dem im Medienkanal 12 zu transportierenden Medium (d. h. es sollte vermieden werden, dass das Mantelmaterial chemisch oder auf sonstige Weise mit dem Medium reagiert oder von diesem angegriffen wird) und andererseits ausreichende Isolationseigenschaften (d. h. der Mantel 10 sollte eine geeignete elektrisch isolierende Matrix für die Leiterelemente 14 bilden) zu Grunde. Im Ausführungsbeispiel sind die Leitungselemente 14, beispielsweise Kupfer- und/oder Aluminiumdrähte, -kabel oder -litzen, in den Mantel 10 eingegossen bzw. beim Extrudieren eingelegt; unter geeigneter Isolierung können sie aber auch außenseitig am Mantel 10 angeordnet sein. Hinsichtlich der elektrischen Eigenschaften kann der Verbindungsschlauch 4 somit als Kabelstrang 4a angesehen werden, bei dem in einem Mantel 10 eine Anzahl von elektrischen Leitungselementen 14 geführt sind.

In einem endseitigen Bereich weist der Mantel 10 des Verbindungsschlauchs 4 für jedes der Leiterelemente 14 jeweils eine Ausbuchtung 16 auf, in der das jeweilige Leiterelement 14 vom Mantelmaterial frei gehalten ist. Im Bereich der jeweiligen Ausbuchtung 16 ist das jeweilige Leiterelement 14 somit unisoliert und damit elektrisch kontaktierbar; gemäß der nachfolgenden Beschreibung ist somit die elektrische Verbindung des jeweiligen Leiterelements 14 mit einem passenden Kontaktstift ermöglicht.

Gemäß einer als eigenständig erfinderisch angesehenen Ausführungsform kann der Verbindungsschlauch im Querschnitt die in FIG. 4a gezeigte Kontur aufweisen. Der Mantel 10 bildet dabei zwei im Wesentlichen zwei parallele Seitenflächen 17 aus, die vom Benutzer in der Art von Griffflächen gefasst werden können. Die Seitenflächen 17 sind in ihrem ersten Endbereich über eine gerundete Kontur 18 miteinander verbunden, wohingegen der die Seitenflächen 17 verbindende zweite Endbereich eine Ecke oder Kante 19 ausbildet. Durch diese Konturierung ist es bei der Montage oder auch für den Benutzer besonders einfach und zuverlässig möglich, allein auf haptischer Basis, also durch Fühlen und ohne den Verbindungsschlauch ansehen zu müssen, die Lage und Orientierung des Verbindungsschlauchs 4 und der in diesen integrierten Leitungselemente 14 zu erfassen und zu erkennen. Gerade bei einer Ausgestaltung, bei der die korrekte individuelle Zuordnung und Handhabung der Leiterelemente 14 wichtig ist, wie beispielsweise zur Einhaltung einer vorgegebenen elektrischen Polarität der Leiterelemente 14 bei der Montage oder auch Bedienung des Gesamtsystems, erhöht diese oder eine ähnliche Querschnittsform die Bedienfreundlichkeit und Zuverlässigkeit des Systems ganz erheblich. In einer alternativen, ebenfalls besonders bevorzugten Ausführungsform kann der Mantel 10 des Verbindungschlauchs 4' auch die in der Querschnittsdarstellung in FIG. 4b dargestellte Kontur aufweisen. Dabei ist der Verbindungsschlauch 4' bzw. der dessen Außenbereich bildende Mantel 10 mit ovalem, oder allgemeiner nicht rundem, Querschnitt ausgeführt. Damit wird ebenfalls in besonders vorteilhafter Weise erreicht, dass bei der Montage auf rein haptische Weise, also lediglich über das Griffgefühl, eine Information über die räumliche Orientierung und somit beispielsweise über eine korrekte Einbaulage erhältlich ist.

Die Ausgestaltung des Verbindungsschlauchs 4, 4' in der beschriebenen Art, im Allgemeinen als Schlauch oder Kabelstrang 4a mit einem von einem Mantel 10 umgebenen Medienkanal 12, wobei im oder am Mantel 10 eine Anzahl von elektrischen Leitungselementen 14 angeordnet sind, und/oder dessen Verwendung in einem Behandlungssystem 1 der beschriebenen Art, werden als eigenständig erfinderisch angesehen. Die Kontur des Querschnitts wie in Fig. 4a dargestellt wird darüber hinaus als eigenständig erfinderisch angesehen für Schläuche im allgemeinen oder auch elektrische Leitungen, Kabel oder Kabelstränge, wobei in allen diesen Fällen aufgrund der Querschnittskontur die haptische Erfassung der räumlichen Orientierung und damit beispielsweise einer korrekten Polung oder Orientierung ermöglicht ist.

Zur elektrischen Verbindung der Leitungselemente 14 mit der elektrischen Versorgungseinheit 8 ist diese mit einem Kontaktierungssystem 20 versehen. Die Kontaktierung der Leitungselemente 14 ist dabei insbesondere hinsichtlich der für medizinische Anwendungen erforderlichen und zwingend einzuhaltenden Zuverlässigkeit einerseits, andererseits aber auch für eine vergleichsweise einfache Bedienbarkeit ausgeführt. Dazu umfasst das in FIG. 5 ausschnittsweise vergrößert dargestellte Kontaktierungssystem 20 ein zur Aufnahme eines Teilabschnitts des Kabelstrangs 4a vorgesehenes Anschlussmodul 22, das in eine korrespondierende, an der elektrischen Versorgungseinheit 8 angebrachte Kontaktdose 24 einsteckbar oder einsetzbar ist.

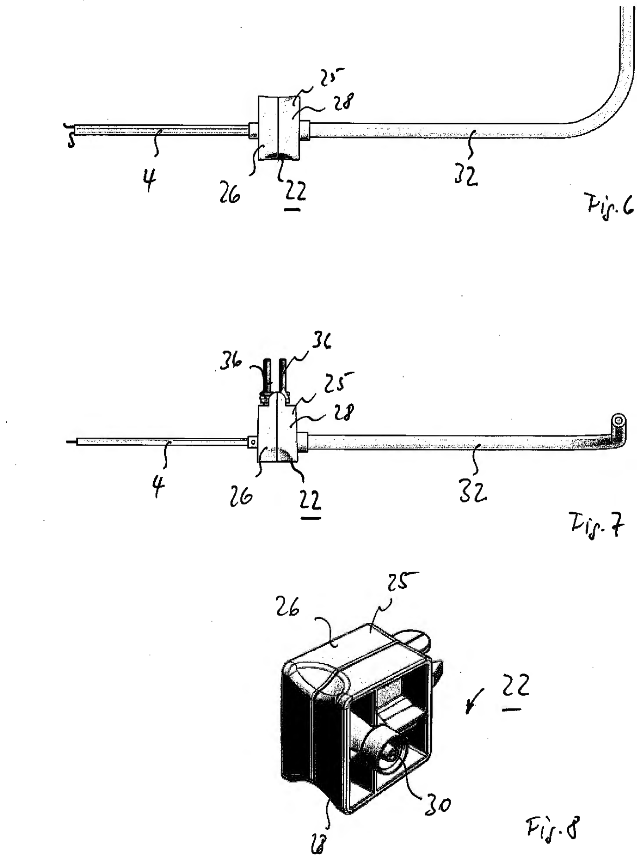

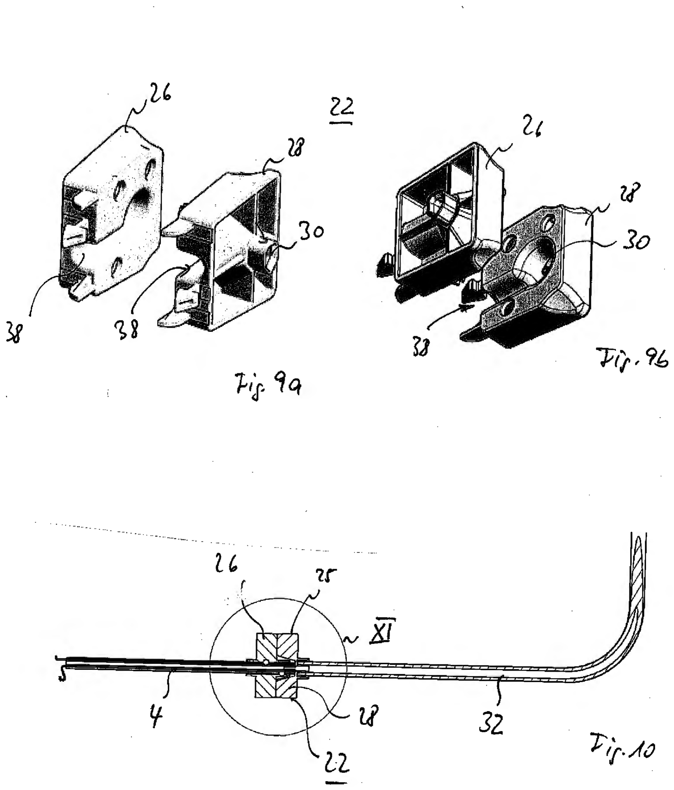

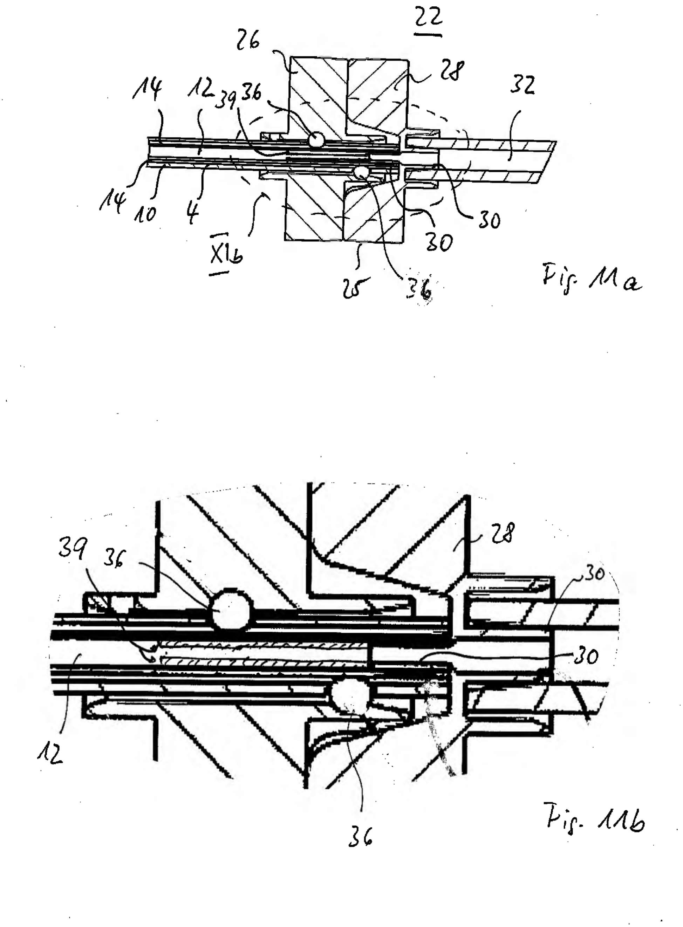

Das Anschlussmodul 22 wird hinsichtlich der nachfolgend näher erläuterten Ausgestaltungen ebenfalls als eigenständig erfinderisch angesehen. Es ist in den FIGs. 6 - 11 näher dargestellt, und zwar in den FIGs. 6 und 7 in an den Verbindungsschlauch 4 angeschlossenem Zustand in seitlicher Ansicht und in Draufsicht, in FIG. 8 in perspektivischer Ansicht, in FIG. 9 in Explosionsdarstellung seines Außengehäuses 25, und in den FIGs. 10 und 11 im Längsschnitt. Wie diesen Darstellungen entnehmbar ist, wird das Außengehäuse 25 des Anschlussmoduls 22 im Ausführungsbeispiel von zwei zusammensteckbaren Gehäusehälften 26, 28 gebildet, wobei der Verbindungsschlauch 4 durch die erste Gehäusehälfte 26 in das Gehäuseinnere eingeführt und mit seinem Medienkanal 12 auf eine im Gehäuseinneren an der zweiten Gehäusehälfte 28 angeordnete Schlauchtülle oder einen Schlauchstutzen 30 aufgesteckt ist. Der Verbindungsschlauch 4 ist somit mit seinem endseitigen Teilabschnitt innerhalb des durch die Gehäusehälften 26, 28 gebildeten Außengehäuses 25 des Anschlussmoduls 22 fixiert. Die Schlauchtülle 30 ist ihrerseits durch die zweite Gehäusehälfte 28 hindurchgeführt und an der Außenseite des Gehäuses mit einem Versorgungsschlauch 32 verbunden, der seinerseits über eine Pumpe 34 an den Versorgungsbehälter 6 angeschlossen ist.

In seinem Endbereich, unmittelbar benachbart zur Schlauchtülle 30 und innerhalb des Außengehäuses 25, sind die Ausbuchtungen 16 des Verbindungsschlauchs 4 positioniert. An dieser Stelle ist der durch den Verbindungsschlauch 4 gebildete Kabelstrang 4a somit von der Außenseite her abisoliert, d. h. der Mantel 10 ist dort insoweit im Au-ßenbereich entfernt oder verdünnt, dass die Leiterelemente 14 in diesem Bereich frei liegen, keine Isolierung aufweisen und somit elektrisch kontaktierbar sind. Zur Kontaktierung dieser freigelegten Bereiche der Leiterelemente 14 sind dabei zwei geeignete Kontaktierungselemente mit einer zur Kontaktierung geeigneten Geometrie, beispielsweise einem Radius, einem Konus oder einer Abschrägung, vorgesehen. Im Ausführungsbeispiel sind diese Kontaktierungselemente als Kontaktstifte 36 ausgestaltet, die von der Seite her relativ zur Längsrichtung des Kabelstrangs 4a gesehen quer durch das Außengehäuse 25 des Anschlussmoduls 22 hindurchgeführt sind. Im Ausführungsbeispiel sind diese Kontaktstifte 36 fest in der Kontaktdose 24 montiert und in der elektrischen Versorgungseinheit 8 geeignet mit den dort vorgesehenen Versorgungskomponenten wie beispielsweise Strom- oder Spannungsquellen verbunden. Korrespondierend zu den Kontaktierungsstiften 36 weisen die Gehäusehälften 26, 28 geeignete Durchführungslöcher 38 für die Kontaktstifte 36 auf. Auf diesem Wege und mittels der genannten Komponenten wird im Kabelstrang der Medienkanal von den Stromleitern auf- oder abgespalten.

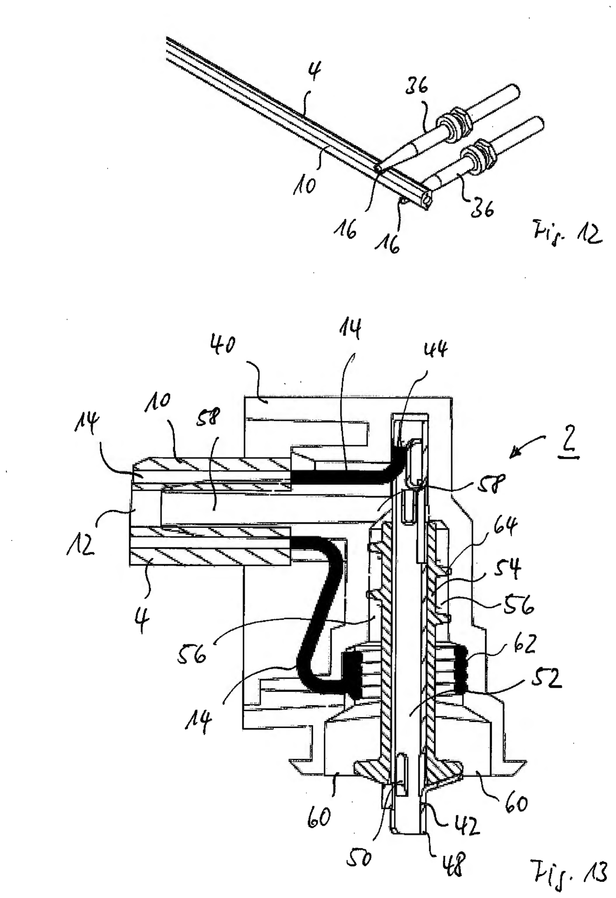

Beim Einstecken des mit dem endseitigen Bereich des Verbindungsschlauchs 4 versehenen Anschlussmoduls 22 in die Kontaktdose 24 werden die Kontaktstifte 36 somit in die Durchführungslöcher 38 eingebracht und ragen durch diese hindurch quer zur Längsrichtung des Kabelstrangs 4a in das Innere des durch die Gehäusehälften 26, 28 gebildeten Außengehäuses 25. Dort können sie den durch den Verbindungschlauch 4 gebildeten Kabelstrang 4a kontaktieren. Dieser wird beim Einstecken in einer Orientierung quer zu den Kontaktstiften 36 derart zwischen diese eingeschoben, dass jedes der außenseitig angeordneten, abisolierten Leiterelemente 14 jeweils einem der Kontaktstifte 36 zugewandt ist. Die Kontaktstifte 36 sind dabei relativ zueinander derart beabstandet und im Übrigen innerhalb des Gehäuses derart positioniert angeordnet, dass der Verbindungsschlauch 4 mit seinem abisolierten Endbereich gerade, vorzugsweise leicht klemmend, zwischen die Kontaktstifte 36 passt und somit ein zuverlässiger elektrischer Kontakt zwischen dem jeweiligen Kontaktstift 36 und dem zugeordneten Leiterelement 14 gebildet wird. Das Kontaktierungsprinzip ist in der perspektivischen Darstellung gem. FIG. 12 verdeutlicht, in der unter Weglassung der weiteren Komponenten lediglich die - an sich im Ausführungsbeispiel fest in der Kontaktdose 24 montierten - Kontaktstifte 36 und der endseitig zwischen diesen liegende Verbindungsschlauch 4 gezeigt sind. Deutlich ist dabei erkennbar, wie die Kontaktstifte 36 jeweils in die ihnen zugeordnete Ausbuchtung 16 im Mantel 10 des Verbindungsschlauchs 4 eingreifen und dabei das jeweils innenliegende Leiterelement 14 kontaktieren.

Im solchermaßen eingesteckten Zustand des endseitigen Teils des Verbindungsschlauchs 4 üben die Kontaktstifte 36 einen gewissen, vorzugsweise federnden, Druck auf die im Verbindungsschlauch 4 geführten Leiterelemente 14 aus, insbesondere um eine zuverlässige und stabile elektrische Kontaktierung in diesem Bereich zu gewährleisten. Aufgrund der Materialwahl des Schlauchmaterials könnte dies allerdings zu einem ungewollten Zusammendrücken des Schlauchs im Kontaktbereich führen, wenn die Leiterelemente 14 infolge der Kontaktierung dem Druck nachgeben und nach innen hin ausweichen. Dies könnte einerseits den elektrischen Kontakt schwächen und andererseits zu einer unerwünschten Verengung des Innenquerschnitts des Verbindungsschlauchs 4 führen, so dass der Medienstrom beeinträchtigt wäre. Um dem entgegenzuwirken, ist der Medienkanal 12 des Verbindungssschlauchs 4 in eigenständig erfinderischer Ausgestaltung in seinem endseitigen Bereich unmittelbar angrenzend zum eigentlichen Endbereich, der in montiertem Zustand die Schlauchtülle 30 aufnimmt, mit einem intergrierten Versteigungselement, bevorzugt einem Innenröhrchen 39 aus Metall, Keramik oder einem anderen geeigneten Material, versehen, wie dies in Fig. 11 und insbesondere im vergrößerten Ausschnitt in Fig. 11b erkennbar ist. Das bevorzugt vergleichsweise dünnwandig ausgeführte Innenröhrchen ist dabei insbesondere derart endeitig innerhalb des Medienkanals 12 positioniert, dass es mit seiner endwärts gerichteten Stirnseite an die Endfläche der Schlauchtülle 30 anstößt.

Vorzugsweise wird der Verbindungsschlauch 4 mit seiner Längsrichtung im wesentlichen senkrecht zur Längsrichtung der Kontaktstifte 36 zwischen diese eingeschoben, wie dies im Ausführungsbeispiel gem. FIG. 12 auch gezeigt ist. Durch die im Wesentlichen kreuzende Ausrichtung der Längsrichtungen der Kontaktstifte 36 einerseits und der Leitungselemente 14 im Kabelstrang 4a andererseits ist sichergestellt, dass weder eine örtliche Verschiebung des Verbindungsschlauchs 4 oder Kabelstrangs 4a in seiner Längsrichtung noch eine Ungenauigkeit der Einschiebetiefe des Verbindungsschlauchs 4 in Längsrichtung der Kontaktstifte 36 die Ausbildung jeweils eines Kontaktpunkts zwischen jeweils einem der Leiterelemente 14 und einem der Kontaktstifte 36 gefährdet oder beeinträchtigt. Ein solches System der "kreuzenden Leitungselemente" ist somit besonders unempfindlich gegen Ungenauigkeiten bei der Endmontage und somit besonders betriebssicher und einfach im Zusammenbau.

Alternativ könnten die Kontaktstifte 36 selbstverständlich auch fest mit dem Anschlussmodul 22 verbunden sein, wobei dementsprechend und korrespondierend geeignet Kontakt- oder Aufnahmelöcher in der Art einer Steckdose in der Kontaktdose 24 vorzusehen wären.

Die Ausgestaltung des Kontaktierungssystems 20 in der beschriebenen Art, im Allgemeinen als Kombination eines Anschlussmoduls 22 mit einer Kontaktdose 24, bei dem ein elektrischer Kontakt zur im äußeren Mantelbereich eines Schlauchs oder Kabels geführten Leiterelementen 14 über quer zu diesen, bevorzugt im Wesentlichen senkrecht zu diesen, ausgerichteten Kontaktstiften 36 erfolgt, und/oder dessen Verwendung in einem Behandlungssystem 1 der beschriebenen Art werden als eigenständig erfinderisch angesehen.

Gemäß dem in der WO 2014/075755 A1, der WO 2014/122187 A1, der WO 2014/122188 A1 und/oder der WO 2016/023998 A1 beschriebenen Konzept ist das Behandlungssystem 1 dafür ausgelegt, den zu Reinigungszwecken des behandlungsbedürftigen Bauteils vorgesehenen Stromfluss unter Nutzung der Leitfähigkeit der vorgesehenen Behandlungsflüssigkeit gezielt durch die behandlungsbedürftige Oberfläche hindurch zu leiten. Der Behandlungskopf 2 ist dabei einerseits nach dem Auslegungsprinzip aufgebaut, dass der elektrische Strom dem behandlungsbedürftigen Bauteil zugeführt und dieses als Elektrode verwendet werden kann. Andererseits ist zur Bildung eines Gegenpols oder der Gegenelektrode die Nutzung der elektrischen Leitfähigkeit der über den Behandlungskopf 2 zugeführten Behandlungsflüssigkeit vorgesehen.

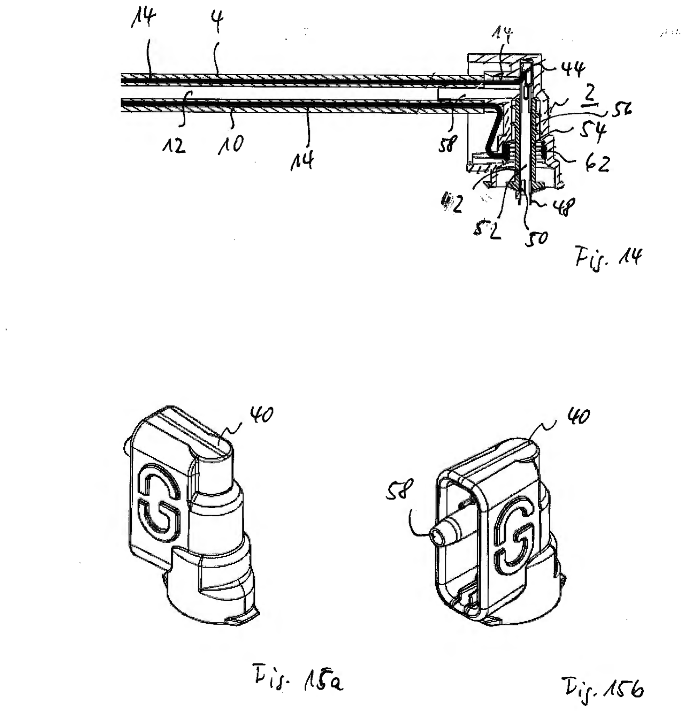

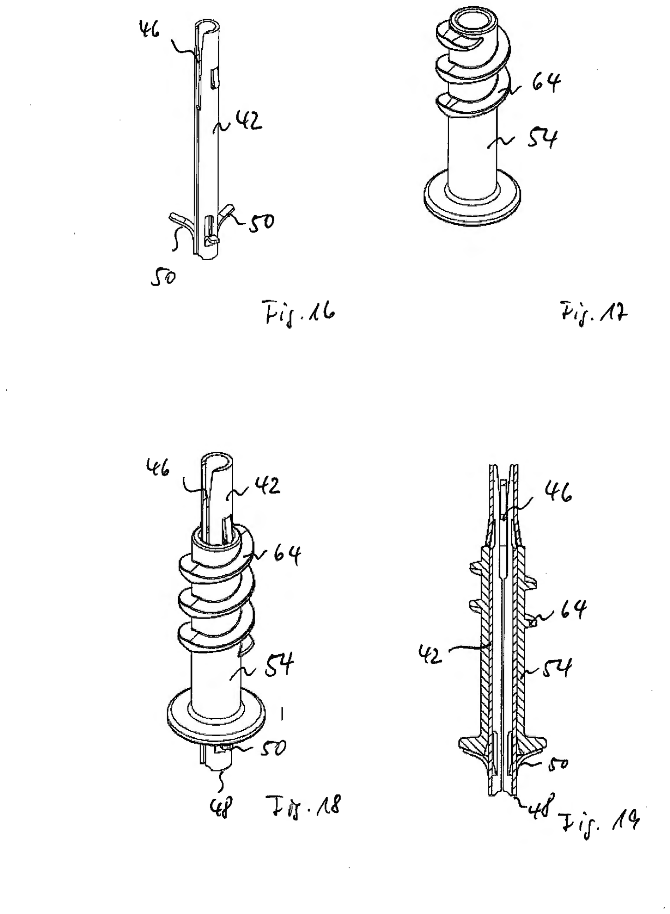

Dazu weist der Behandlungskopf 2 einen Aufbau auf, wie er im Schnitt in den FIGs. 13 (vergrößert) und 14 gezeigt ist. Innerhalb des in den FIGs. 15a und 15b in zwei verschiedenen Perspektiven dargestellten Außengehäuses 40, in das das freie Ende des Verbindungsschlauchs 4 eingeführt ist, ist zum einen zur Bildung eines ersten Elektrodenanschlusses, bevorzugt des Kathodenanschlusses, ein Verbindungsstift 42 angeordnet. Der in FIG. 16 vergrößert dargestellte Verbindungsstift 42 ist dabei elektrisch gut leitfähig und bevorzugt aus einem Metall, ganz besonders bevorzugt aus Titan, hergestellt. Ganz besonders bevorzugt und im Hinblick auf besonders gering gehaltene Herstellkosten ist der Verbindungsstift 42 aus einem gestanzten gebogenen und/oder gerollten Metallblech, besonders bevorzugt aus Titanblech, hergestellt. An seinem im eingebauten Zustand "oberen Ende" innerhalb des Außengehäuses ist der Verbindungsstift 42 mit einem der Leiterelemente 14 des Verbindungsschlauchs 4 verbunden, so dass er bei an die elektrische Versorgungseinheit 8 angeschlossenem Verbindungsschlauch 4 unmittelbar elektrisch ansteuerbar und zur Herstellung einer elektrodischen Verbindung nutzbar ist.

In ebenfalls als eigenständig erfinderisch angesehener besonders bevorzugter Ausgestaltung ist der Verbindungsstift 42 dabei, besonders bevorzugt in Kombination mit dem zur Herstellung dieser elektrischen Verbindung vorgesehenen Ende 44 des entsprechenden Leiterelements 14, zur Sicherstellung einer besonders zuverlässigen elektrischen Kontaktierung ausgeführt. Dazu ist zum einen, wie dies der Schnittdarstellung in FIG. 13 gut entnehmbar ist, das Ende 44 des Leiterelements gebogen ausgeführt, so dass sich der Endbereich federnd an das obere Ende des Verbindungsstifts 42 anlegen kann. Zudem weist der Verbindungsstift 42 in seinem oberen Endbereich eine im Ausführungsbeispiel V-artig ausgeführte Ausnehmung 46 auf, in die das Ende 44 - vorzugsweise klemmend - eingeschoben werden kann.

An seinem freien, im eingebauten Zustand "unteren" Ende 48 ist der Verbindungsstift 42 geeignet zum Aufsetzen auf das behandlungsbedürftige Bauteil ausgeführt. Besonders bevorzugt ist das Behandlungssystem 1 zur Behandlung inserierter medizinischer Implantate im Allgemeinen vorgesehen. Im Ausführungsbeispiel ist das Behandlungssystem 1 gezielt für die ganz besonders bevorzugte Bearbeitung oder Aufbereitung inserierter Dentalimplantate ausgelegt. Dementsprechend ist der Verbindungsstift 42 im Ausführungsbeispiel geeignet zum Aufstecken auf ein inseriertes Dentalimplantat ausgelegt. Falls dieses eine Innenverbindung für ein zugeordnetes Aufbauteil oder Abutment besitzt, so ist das freie Ende 48 bevorzugt an die Abmessungen dieser Innenverbindung angepasst, so dass es passend in das zu behandelnde Implantat eingesteckt werden kann. Zur Sicherstellung eines besonders zuverlässigen elektrischen Kontakts des Verbindungsstifts 42 mit dem behandlungsbedürftigen Bauteil ist der Verbindungsstift 42 zudem an seinem freien Ende mit einer Anzahl von ausgebogenen Federstegen 50 versehen. Diese stellen beim Aufsetzen auf ein passendes Dentalimplantat einen innigen elektrischen Kontakt zu diesem her.

Der Verbindungsstift 42 ist vornehmlich zur Herstellung der elektrischen Verbindung mit dem behandlungsbedürftigen Bauteil, insbesondere dem Dentalimplantat, vorgesehen, so dass dieses als Elektrode für den Reinigungsprozess genutzt werden kann. Demzufolge könnte der Verbindungsstift 42 auch als - vorzugsweise metallischer - Vollkörper ausgeführt sein, da eine gute elektrische Leitfähigkeit als das bedeutendste Auslegungskriterium - neben beispielsweise biologischer Verträglichkeit und dergleichen - angesehen wird. Im Ausführungsbeispiel ist jedoch eine besonders bevorzugte Ausführungsform gezeigt, bei der der Verbindungsstift 42 als Hohlkörper in der Art eines Röhrchens ausgeführt ist. Dieser Hohlkörper, erhältlich beispielsweise durch Rollen eines zuvor geeignet gestanzten Metallblechs, bildet einen Innenkanal 52, durch den Behandlungsflüssigkeit in den Innenraum des darunter befindlichen Implantats eingeleitet und dort zu Reinigungszwecken beispielsweise in der Art einer Spülung durch eine Spüllösung genutzt werden kann. Zusätzlich kann auf diese Weise auch im Innenbereich des Implantats eine Reinigung stattfinden.

Wie der Darstellung in FIG. 13 weiterhin entnehmbar ist, ist der Verbindungsstift 42 in einem ihn umgebenden Isolatorkörper 54 angeordnet, insbesondere in diesen eingesteckt. Der in FIG. 17 separat dargestellte Isolatorkörper 54 ist dabei bevorzugt aus einem geeignet gewählten Kunststoff hergestellt, bevorzugt durch Spritzguss. FIG. 18 zeigt den in den Isolatorkörper 54 eingesteckten Verbindungsstift 42.

Der Isolatorkörper 54 ist, wie dies insbesondere aus der Darstellung in FIG. 13 deutlich wird, seinerseits innerhalb des Außengehäuses 40 des Behandlungskopfes 2 von einem Hohlraum 56 umgeben, der über einen in den Behandlungskopf 2 integrierten Medienkanal 58 mit dem Medienkanal 12 des Verbindungsschlauchs 4 verbunden ist. Über den Medienkanal 58 kann somit Behandlungsflüssigkeit aus dem Medienkanal 12 des Verbindungsschlauchs 4 in den Hohlraum 56 im Behandlungskopf 2 eingebracht werden. In seinem "unteren", dem freien Ende und dem behandlungsbedürftigen Bauteil zugewandten Bereich des Behandlungskopfs 2 weitet sich der Hohlraum 56 auf und bildet rund um den zentral geführten Verbindungsstift 42 und den diesen umgebenden Isolatorkörper 54 eine ringförmige Ausströmfläche 60, durch die die zugeführte Behandlungsflüssigkeit austreten und dem behandlungsbedürftigen Bauteil zuströmen kann.

Zur Bildung eines Gegenpols oder der Gegenelektrode zur Durchführung des elektrolytischen Aufbereitungs- und Reinigungskonzepts, wie es dem Grunde nach aus der WO 2014/075755 A1, der WO 2014/122187 A1, der WO 2014/122188 A1 und der WO 2016/023998 A1 bekannt ist, ist wie bereits erwähnt die Nutzung der elektrischen Leitfähigkeit der über den Behandlungskopf 2 in den Hohlraum 56 und von dort dem behandlungsbedürftigen Bauteil zugeführten Behandlungsflüssigkeit vorgesehen. Um dies zu ermöglichen, ist im Hohlraum 56 eine Elektrode 62 angeordnet, die elektrisch mit dem anderen Leiterelement 14 des Verbindungsschlauchs 4 verbunden ist. Die Elektrode 62 ist grundsätzlich ringförmig ausgeführt und derart im Hohlraum 56 angeordnet, dass sie von der durchfließenden Behandlungsflüssigkeit umströmt und intensiv benetzt wird. Somit ist bei an die elektrische Versorgungseinheit 8 angeschlossenem Verbindungsschlauch 4 die Behandlungsflüssigkeit im Hohlraum 56 und dementsprechend auch im Bereich unmittelbar benachbart zur Ausströmfläche 60 über die elektrische Versorgungseinheit 8 elektrisch ansteuerbar und zur Herstellung einer elektrodischen Verbindung nutzbar.

Die Elektrode 62 ist einerseits für einen besonders guten elektrischen Kontakt mit der sie umströmenden Behandlungsflüssigkeit ausgelegt. Dies wird in als eigenständig erfinderisch angesehener Ausführung einerseits durch die Formgebung der Elektrode 62 erreicht oder zumindest begünstigt: durch die Ringform ist bereits grundsätzlich eine gleichmäßige und großflächige Kontaktierung der Flüssigkeit ermöglicht. Zusätzlich und bevorzugt weist die Elektrode 62 aber auch noch eine Oberflächenkontur wie beispielsweise eine Riffelung oder eine Wellenform auf. Eine solche Struktur vergrößert den Strömungsweg der Flüssigkeit entlang der Oberfläche und somit die effektive Kontaktfläche, und gegebenenfalls kann sie auch Turbulenzen oder Verwirbelungen in der Flüssigkeitsströmung erzeugen, die einen innigen Kontakt zur Oberfläche noch weiter begünstigen. Des Weiteren wird ein besonders guter elektrischer Kontakt zwischen Elektrode 62 und Flüssigkeit auch durch eine geeignete und besonders bevorzugte Materialwahl weiter begünstigt.

Vorteilhafterweise ist die Oberfläche der Elektrode 62 aus einem gut leitfähigen und weiter bevorzugt auch aus einem physiologisch inerten und für den menschlichen Körper gut verträglichen Material, insbesondere Metall, gefertigt, besonders bevorzugt aus Gold, Platin, Magnesium oder dotiertem Diamant. Die Elektrode 62 kann dabei in der Art eines Vollkörpers vollständig aus einem derartigen Material bestehen, oder sie kann alternativ auch von einem beschichteten Trägerkörper gebildet sein, wobei die Oberflächenbeschichtung aus einem der genannten Materialien besteht.

Andererseits ist die Elektrode 62 aber auch für eine vergleichsweise einfache Montierbarkeit des Behandlungskopfs 2 besonders günstig ausgelegt. Dabei liegen als Auslegungskriterien bevorzugt zugrunde, dass im Sinne einer einfachen Montage der mit dem Isolatorkörper 54 versehene Verbindungsstift 42 einfach über das untere Ende des Behandlungskopfes 2 in dessen Außengehäuse 40 eingeschoben werden können sollte, und dass der eingebrachte Verbindungsstift 42 im Außengehäuse 40 nach seiner Montage vergleichsweise festen Halt und einen guten Sitz finden sollte. Um beide Kriterien gleichermaßen erfüllen zu können, ist die Elektrode 62 vorteilhafterweise derart ausgeführt, dass sie federnd nachgebend eine vorübergehende Aufweitung ihres lichten Innenquerschnitts zulässt.

Zu diesem Zweck kann die Elektrode 62 in einer bevorzugten Ausführungsform als durchbrochener Ring oder Sprengring oder in der Art eines geschlitzten Rohrstücks ausgeführt sein. Im Ausführungsbeispiel ist die Elektrode 62 in der ganz besonders bevorzugten Ausführungsform als Feder oder gewickelter Draht gezeigt. Eine solche Bauform bietet einerseits den Vorteil der erwünschten Elastizität bei vorübergehender Aufweitung, und andererseits ist die Oberfläche bauartbedingt wellig oder geriffelt.

Durch die Bauweise des Behandlungskopfes 2 ist erreicht, dass der zu Behandlungs- und Reinigungszwecken angelegte elektrische Strom durch die von den Bakterien befallene Oberflächenzone des behandlungsbedürftigen Bauteils hindurch- und von dort aus weitgehend unmittelbar, also insbesondere ohne "Umwege" über weiteres Körpergewebe oder dergleichen, zur als Kontaktfläche dienenden Ausströmfläche 60 fließen kann. Die Medienkanäle 12, 58 einschließlich der darin geführten, elektrisch leitfähigen Behandlungsflüssigkeit und den entsprechenden Anschlusselementen bilden somit im Ausführungsbeispiel ein zweites, einen elektrischen Strompfad zum eigentlichen Leiterelement 14 im Verbindungsschlauch 4 bildendes Leiterelement.

Um übermäßige Leckagen bei der Ausbringung der Behandlungsflüssigkeit zu vermeiden oder zumindest zu reduzieren, ist im Bereich der Ausströmfläche 60 und somit am "freien" Ende des Behandlungskopf ein dessen Mündungsbereich umgebender Schwamm vorgesehen.

Im Ausführungsbeispiel ist auch die Beaufschlagung des Innenkanals 52 des Verbindungsstiftes 42 mit Behandlungsflüssigkeit vorgesehen. Um dabei dem Problem eines elektrischen Kurzschlusses zwischen den beiden Elektroden, einerseits gebildet durch den Verbindungstift 42 und andererseits gebildet durch die von der Behandlungsflüssigkeit umströmte Elektrode 62, entgegenzuwirken, ist der im Hohlraum 56 platzierte Isolatorkörper 54 an seinem Außenbereich in als eigenständig erfinderisch angesehener Ausführung mit einem umlaufenden Gewinde 64 versehen, wie dies insbesondere aus der vergrößerten Darstellung in FIG. 17 deutlich wird. Bei in den Hohlraum 56 eingebrachtem Isolatorkörper 54 schließt dieses Gewinde 64 möglichst bündig mit der umgebenden Innenwand des Hohlraums 56 ab. Dadurch bildet das Gewinde 64 einen verlängerten Strömungsweg für die Behandlungsflüssigkeit, da diese, geleitet durch das Gewinde 64, spiralförmig um den Isolatorkörper 54 herumströmen muss. Durch diese künstliche Verlängerung des Strömungswegs wird entsprechend auch die elektrische Wegstrecke in der Behandlungsflüssigkeit im Hohlraum 56 entsprechend verlängert, was deren elektrischen Widerstand entsprechend vergrößert. Auf diese Weise ist es möglich, einen Kurzschluss- oder Fehlstrom "nach oben hin", also zum Zuströmbereich in den Verbindungsstift 42, so gering wie möglich zu halten, da dieser Fehlstrom für den beabsichtigten Reinigungseffekt nicht zur Verfügung stünde.

In den FIGs. 18 und 19 ist das aus dem Verbindungsstift 42 und dem Isolatorkörper 54 zusammengesetzte Ensemble in perspektivischer Darstellung (FIG. 18) und im Längsschnitt (FIG. 19) gezeigt.

Die vorgenannten Ausgestaltungen und Einzelteile, insbesondere der Verbindungsschlauch 4 in der beschriebenen Art, der Verbindungsstift 42, der Isolatorkörper 54 mit umlaufendem Außengewinde 64, die Elektrode 62 mit den genannten Auslegungskriterien, die Ausgestaltung des Behandlungskopfes 2, jeweils einzeln oder in Kombination miteinander, sowie deren Verwendung, jeweils einzeln oder in Kombination miteinander, in einem Behandlungssystem 1 der genannten Bauweise, werden ausdrücklich als eigenständig erfinderisch angesehen.

Die zur Verwendung in dem Behandlungssystem 1 vorgesehene Behandlungsflüssigkeit ist im Hinblick auf die bereits aus den WO 2014/075755 A1, der WO 2014/122187 A1, der WO 2014/122188 A1 und der WO 2016/023998 A1 bekannten Aspekte geeignet gewählt und zusammengesetzt. Die Auswahl und Zusammensetzung der Grundbestandteile der Behandlungsflüssigkeit ist dabei insbesondere im Hinblick auf die beabsichtigte Wirkungsweise, d. h. der Aufbringung eines elektrischen Stroms im Raumbereich der behandlungsbedürftigen Oberfläche, vorgenommen, wobei insbesondere sichergestellt ist, dass eine für diesen Zweck ausreichend hohe elektrische Leitfähigkeit in der Behandlungsflüssigkeit vorliegt. Diese soll insbesondere durch eine ausreichend hoch gewählte Ionendichte in der Behandlungsflüssigkeit sichergestellt werden. Dazu ist als ein Grundbestandteil der Behandlungsflüssigkeit ein Metallsalz vorgesehen, bevorzugt in wässriger Lösung. Besonders bevorzugt wird eine Lösung mit dem Metallsalz Natriumformiat verwendet. Das Metallsalz liefert die Ionen für den Stromtransport, und zudem können die nach der jeweiligen Elektrodenreaktion entstehenden Umsetzungsprodukte auch noch geeignete biochemische Wirkungen aufweisen. Durch die gezielte Wahl einer ausreichend hohen elektrischen Leitfähigkeit soll bei einer Durchführung des Reinigungsverfahrens an einem inserierten Implantat sichergestellt werden, dass der Stromfluss durch die Behandlungsflüssigkeit und damit durch die behandlungsbedürftigen Teile und Komponenten, nicht aber durch das Körpergewebe des Patienten erfolgt, so dass eine Gefährdung des Patienten durch einen ungewollten Stromfluss durch Weichgewebe, Knochen, Blut und/oder andere Körpermaterialien minimiert werden kann. Die elektrische Leitfähigkeit der Behandlungsflüssigkeit sollte dabei möglichst ein Vielfaches der elektrischen Leitfähigkeit von Blut, Knochen, Weichgewebe, Fettgewebe oder anderen Körpermaterialien aufweisen.

Demzufolge werden bei der Auswahl und Zusammensetzung der Grundbestandteile für die Behandlungsflüssigkeit insbesondere folgende Leitfähigkeitswerte berücksichtigt (die elektrische Leitfähigkeit σ wird dabei in der üblichen Einheit mS/cm angegeben):

| | |
|---|---|
| Haut: | 0,03 - 0.1 mS/cm |
| Knochen: | 0,06 - 0,2 mS/cm |
| Fettgewebe: | 0,20 - 1,0 mS/cm |
| Muskelgewebe: | 0,80 - 2,5 mS/cm |
| Blut: | ca. 6,7 mS/cm |
| andere Körperflüssigkeiten: | ca. 15 mS/cm |

Um das Gefährdungspotential für den Patienten geeignet gering zu halten und den Stromfluss auf die erwünschten Regionen zu begrenzen, sollte die elektrische Leitfähigkeit daher mindestens das Doppelte, bevorzugt das Fünffache, besonders bevorzugt das Zehnfache der Leitfähigkeit sonstiger Körperflüssigkeiten betragen. Daher sollte die elektrische Leitfähigkeit der Behandlungsflüssigkeit einen Wert von mindestens 30 mS/cm, vorzugsweise mindestens 75 mS/cm und besonders bevorzugt mindestens 150 mS/cm aufweisen. Im Vergleich zu Blut bedeutet dies, dass die elektrische Leitfähigkeit der Behandlungsflüssigkeit vorzugsweise mindestens etwa das Fünffache, bevorzugt mindestens etwas das Zehnfache und besonders bevorzugt mindestens etwa das Zwanzigfache der Leitfähigkeit von Blut beträgt. Messungen haben ergeben, dass beim Einsatz einer solchermaßen gewählten Behandlungsflüssigkeit die elektrische Spannung, der das Körpergewebe, das Blut, die Körperflüssigkeiten etc. ausgesetzt werden, geringer als 6 V, vorzugsweise geringer als 3 V, besonders bevorzugt kleiner als 1,5 V ist. Damit können Schädigungen für den Patienten aufgrund der gering gehaltenen Spannungen sicher ausgeschlossen werden. Zur Einhaltung einer solchen Leitfähigkeit ist insbesondere die Ionenkonzentration in der Behandlungsflüssigkeit und in den sie bildenden Grundbestandteilen ausreichend hoch gewählt; hierfür können Laugen, Säuren, Salze und/oder andere ionenbildende Stoffe oder Stoffverbindungen zum Einsatz kommen.

Bei der Auswahl und Zusammensetzung der Grundbestandteile der Behandlungsflüssigkeit ist in besonderem Maße berücksichtigt, dass die reinigende oder biofilmablösende Wirkung der elektrolytischen Behandlung einer kontaminierten Implantatoberfläche auf einer Kombination mehrerer Ursachen beruht, die möglichst ergänzend zueinander nutzbar gemacht werden sollten. Zum einen können sich beim Stromfluss durch den Elektrolyten bevorzugt im Bereich der Elektroden Gase oder Gasblasen bilden, welche eine abhebende (mechanische) Wirkung auf den Biofilm haben. Die Entstehung dieser Gase erfolgt unmittelbar an der als Elektrode dienenden Implantatoberfläche und somit zwischen dieser und dem Biofilm. Die dabei entstehenden Gasblasen beeinflussen mit ihrer Wachstumsgeschwindigkeit und ihrer maximalen Größe den Ablösungsprozess.

Als zweite Ursache für die das Implantat reinigende oder den Biofilm ablösende Wirkung des elektrolytischen Prozesses ist die zersetzende, zerstörende und auflösende Wirkung der elektrolytisch entstehenden Stoffe oder Stoffverbindungen auf die eigentliche Anhaftung des Biofilms an der Implantatoberfläche, also auf den Klebe- oder Verankerungsmechanismus, zu nennen.

Die dritte Ursache für die reinigende oder ablösende Wirkung des elektrolytischen Prozesses basiert auf materialabtragenden Effekten des Implantatmaterials, wobei Bestandteile oder Partikel des eigentlichen Implantats in dessen Oberflächenbereich aus diesem herausgelöst werden.

Die vierte Ursache für die reinigende oder ablösende Wirkung des elektrolytischen Prozesses basiert auf der Oxidschichtbildung metallischer Implantate, welche dies erlauben. Hierbei durchdringen Metallatome des metallischen Grundmaterials die evtl. schon vorhandene Oxidschicht basierend auf der angelegten elektrischen Spannung und reagieren mit Stoffen des Elektrolyts (meist Sauerstoff => Metalloxidbildung). Bei Metallen, welche keine Oxidschicht bzw. keine mechanisch stabile Oxidschicht bilden, können auch nicht-oxydische Stoffverbindungen entstehen (meist Salze), die dann in Lösung gehen.

Die für die Bildung der Behandlungsflüssigkeit vorgesehenen Grundbestandteile sind im Hinblick auf diese Effekte geeignet gewählt und miteinander kombiniert. Zudem ist als grundlegendes Auslegungsziel berücksichtigt, dass keine toxischen oder anderweitig einen Patienten gefährdenden oder für ihn unangenehmen Effekte auftreten sollten, so dass die Behandlungsflüssigkeit auch für einen Einsatz am inserierten Dentalimplantat, also im Patientenmund, geeignet ist. Im Ausführungsbeispiel sind dabei als Grundbestandteile mindestens ein Salz einerseits und eine Säure andererseits, bevorzugt verdünnt mit Wasser, vorgesehen, deren Auswahl und Zusammensetzung sich insbesondere nach den genannten Kriterien richtet. Besonders bevorzugt ist dabei als Säure Phosphorsäure, Zitronensäure, Ameisensäure, Essigsäure, Milchsäure, Kohlensäure oder eine Kombination von diesen vorgesehen. Alternativ oder zusätzlich besonders bevorzugt ist dabei als Salz Natrium-, Calcium-, Aluminium-, Magnesium-, Zinn- oder Kaliumiodid, -chlorid, -nitrat, -carbonat oder -hydrogencarbonat und/oder Ammoniumchlorit, -nitrat oder -iodid oder eine Kombination von diesen vorgesehen. Ganz besonders bevorzugt ist als Metallsalz Natriumformiat.vorgesehen; Natriumformiat ist das Natriumsalz der Ameisensäure mit der Konstitutionsformel Na(HCOO).

Das Behandlungssystem 1 und insbesondere auch dessen elektrische Versorgungseinheit 8 und/oder eine dieser zugeordnete Steuerung ist für eine koordinierte Verfahrensführung in dem Sinne ausgelegt, dass die Einspeisung der Behandlungsflüssigkeit einerseits und die Strombeaufschlagung andererseits abgestimmt aufeinander vorgenommen werden. Dazu kann beispielsweise vorgesehen sein, dass über die Versorgungseinheit 8 eine mit der Bestromung der Leiterelemente 14 koordinierte Ansteuerung der dem Verbindungsschlauch 4 oder dem Vorratsbehälter 6 zugeordneten Pumpe 34 für die Behandlungsflüssigkeit erfolgt. Dies kann automatisiert oder bedarfsweise auch manuell über einen Schalter gesteuert sein. Ein manuell betätigbarer Schalter kann dabei insbesondere direkt im Behandlungskopf 2 angeordnet sein, so dass der Bediener beim Behandeln des Patienten Zugriff auf die Systemsteuerung nehmen kann.

### Bezugszeichenliste

- 1: Behandlungssystem
- 2: Behandlungskopf
- 4, 4': Verbindungsschlauch
- 4a: Kabelstrang
- 6: Vorratsbehälter
- 8: elektrische Versorgungseinheit
- 10: Mantel
- 12: Medienkanal
- 14: Leiterelement
- 16: Ausbuchtung
- 17: Seitenfläche
- 18: Kontur
- 19: Kante
- 20: Kontaktierungssystem
- 22: Anschlussmodul
- 24: Kontaktdose
- 25: Außengehäuse
- 26, 28: Gehäusehälften
- 30: Schlauchtülle
- 32: Versorgungsschlauch
- 34: Pumpe
- 36: Kontaktstift
- 38: Durchgangsloch
- 39: Innenröhrchen
- 40: Außengehäuse
- 42: Verbindungsstift
- 44: Ende
- 46: Ausnehmung
- 48: Ende
- 50: Federsteg
- 52: Innenkanal
- 54: Isolatorkörper
- 56: Hohlraum
- 58: Medienkanal
- 60: Ausströmfläche
- 62: Elektrode
- 64: Gewinde

## Patentansprüche

1. Behandlungskopf (2) zur medienseitigen und elektrischen Kontaktierung eines behandlungsbedürftigen Bauteils, insbesondere eines inserierten Dentalimplantats, mit einem Außengehäuse (40) , in dem ein in einem ihn umgebenden Isolatorkörper (54) angeordneter, auf das Bauteil aufsteckbarer und somit mit diesem elektrisch verbindbarer Verbindungsstift (42) angeordnet ist, wobei der Isolatorkörper (54) innerhalb des Außengehäuses (40) des Behandlungskopfes (2) von einem medienseitig mit einem Medienkanal (58) verbundenen Hohlraum (56) umgeben ist.

2. Behandlungskopf (2) nach Anspruch 1, dessen Hohlraum (56) sich in einem dem behandlungsbedürftigen Bauteil zugewandten Endbereich des Behandlungskopfs (2) aufweitet und rund um den zentral geführten Verbindungsstift (42) und den diesen umgebenden Isolatorkörper (54) eine ringförmige Ausströmfläche (60) für den Hohlraum (56) durchströmende Behandlungsflüssigkeit bildet.

3. Behandlungskopf (2) nach Anspruch 1 oder 2, dessen Isolatorkörper (54) außenseitig mit einem umlaufenden, einen Strömungsweg für die Behandlungsflüssigkeit bildenden Gewinde (64) versehen ist.

4. Behandlungskopf (2) nach einem der Ansprüche 1 bis 3, in dessen Hohlraum (56) eine Elektrode (62) zur elektrischen Kontaktierung von den Hohlraum (56) durchströmender Behandlungsflüssigkeit angeordnet ist.

5. Behandlungskopf (2) nach Anspruch 4, dessen Elektrode (62) als Feder oder gewickelter Draht ausgeführt ist.

6. Behandlungskopf (2) nach einem der Ansprüche 1 bis 5, dessen Verbindungsstift (42) als Hohlkörper in der Art eines Röhrchens, bevorzugt erhalten durch Rollen eines zuvor gestanzten Metallblechs, ausgeführt ist.
